# EUROPÄISCHE PATENTSCHRIFT

(11) **EP 1 515 758 B1**
(45) Veröffentlichungstag und Bekanntmachung des Hinweises auf die Patenterteilung: **11.03.2009**
(21) Anmeldenummer: 03732486.0
(22) Anmeldetag: 27.05.2003
(51) Int. Cl.: A61L 27/30, A61L 27/56

(54) **OFFENPORÖSE METALLBESCHICHTUNG FüR GELENKERSATZIMPLANTATE SOWIE VERFAHREN ZUR HERSTELLUNG**
OPEN-PORED METAL COATING FOR JOINT REPLACEMENT IMPLANTS AND METHOD FOR PRODUCTION THEREOF
REVETEMENT METALLIQUE A PORES OUVERTS POUR IMPLANTS D'ARTICULATION ARTIFICIELLE ET PROCEDE POUR LE REALISER

(30) Priorität: 27.06.2002 DE 10228746; 17.09.2002 DE 10243101
(43) Veröffentlichungstag der Anmeldung: 23.03.2005
(73) Patentinhaber: Smith & Nephew Orthopaedics AG, 6343 Rotkreuz (CH)
(72) Erfinder: LERF, Reto, CH-5037 Muhen (CH)
(74) Vertreter: Popp, Eugen
(86) Internationale Anmeldenummer: PCT/EP2003/005586
(87) Internationale Veröffentlichungsnummer: WO 2004/002544

(56) Entgegenhaltungen:
- DE-A- 10 022 162
- US-A- 3 855 638
- US-A- 4 542 539
- US-A- 5 456 723

## Beschreibung

Die Erfindung betrifft eine offenporige biokompatible Oberflächenschicht, ein Verfahren zur Herstellung eines offenporigen beschichteten Implantats, ein Implantat sowie die Verwendung einer offenporigen biokompatiblen Oberflächenschicht gemäß den Oberbegriffen der Patentansprüche 1, 7 und 25.

Implantate und insbesondere Gelenkersatzimplantate gewinnen immer mehr an Bedeutung in der restaurativen und kurativen Medizin. Bei zementfreien Gelenkersatzimplantaten steht hierbei die mechanisch stabile Verankerung des Implantats im Knochen im Vordergrund. Diese ist wesentlich für die langfristige Haltbarkeit und Verträglichkeit der Implantate. Bislang kommt es aber hier - bedingt durch eine Lockerung des Implantats - immer wieder zu einer Osteolyse, die durch Abriebpartikel bedingt ist. Diese sogenannte aseptische Lockerung ist klinisch die häufigste Ursache für Revisionsoperationen, beispielsweise an Hüftgelenken. Somit ist eine Reduktion des Abriebs ein Ziel bei der Entwicklung von Gelenkersatzimplantaten. Ein weiteres Ziel ist, die Ausbreitung der Abriebspartikel entlang des Implantats zu verhindern. Hierbei steht im Vordergrund, dass dem Knochen eine optimierte Oberfläche des Gelenkersatzimplantats geboten wird, so dass dieser in das Implantat einwachsen kann. Eine aseptische Lockerung wird auf diese Weise reduziert. Somit ist die Oberflächenstruktur bzw. -beschichtung eines Gelenkersatzimplantats von entscheidender Bedeutung, da durch diese ein Einwachsen des Knochens in das Implantat, respektive in die Implantatoberfläche ermöglicht oder ver- bzw. behindert wird.

Zur Herstellung solcher Oberflächenschichten haben sich bislang poröse Schichten bewährt. Es sind verschiedene Verfahren bekannt, mit denen sich derartige poröse Schichten herstellen lassen. Als Materialien werden hierbei biokompatible Materialien, insbesondere Metalle, wie beispielsweise Titan eingesetzt. Die Oberflächenschichten werden hierbei zum überwiegenden Teil auf Knochenimplantaten angeordnet, so daß deren Langzeitverankerung im Knochen verbessert ist. Die notwendigen porösen Schichten können beispielsweise mittels Sintertechnik erzeugt werden, wobei die Strukturen und die Sinterbedingungen so gewählt werden, dass Hohlräume zwischen den auf die Oberfläche aufgebrachten Metall- bzw. Titanteilchen erhalten bleiben.

V. Galante et al., JBJS, 53A (1971), Seite 101 - 114, beschreibt an dieser Stelle beispielsweise, daß ein Geflecht aus feinen Titandrähten auf ein Substrat gesintert wird. Die beiden US-Patente 3,855,638 und 5,263,986 offenbaren ein Verfahren, bei dem ein Titanpulver aus kugeligen Partikeln verschiedener Größe auf ein Substrat aufgesintert wird. Das US-Patent 4,206,516 verwendet hingegen ein gemahlenes Titanhydridpulver aus kantigen Partikeln, das wiederum auf ein Substrat aufgesintert wird.

Im weiteren ist es möglich, aus einem Gemisch aus thermisch instabilen Platzhaltern und Titanpulver oder aus Platzhaltern und Titanhydridpulver mittels Sintern ein Skelett aus Titan herzustellen. Diesbezügliche Verfahren sind beispielsweise in den Patentschriften US 5,034,186 oder WO 01/19556 beschrieben; ebenso widmet sich ein Verfahren der Firma Intermedics, Austin, USA unter dem Titel "Cancellous Structured Titanium" einer solchen Möglichkeit.

Allen über einen solchen Sinterprozeß hergestellten Titanschichten ist es jedoch inhärent nachteilig, dass die Rauhheit der ehemaligen Titanpartikel oder Titanfasern durch eine Oberflächendiffusion eingeglättet wird. Der Knochen kann zwar in die Poren einwachsen, aber mikroskopisch gesehen, finden die Knochenzellen kaum Halt an der glatten Titanoberfläche. Dieser Nachteil kann umgangen werden, indem die Zeit, in der die Titanteilchen hohen Temperaturen ausgesetzt sind, drastisch reduziert wird. Dies ist z.B. beim Flamm-Spritzprozeß der Fall. Dem Gewinn an Rauhigkeit, der in diesem Verfahren im mikroskopischen Bereich erzielt werden kann, steht jedoch ein gravierender Nachteil gegenüber, da flammgespritzte oder plasmagespritzte Titanschichten praktisch keine nach außen offenen Poren aufweisen und somit ein Einwachsen des Knochens per se verhindern, da die hierfür notwendigen Poren nicht vorhanden sind. Der Lösung dieses Problems hat sich beispielsweise das US-Patent 4,542,539 gewidmet. Weitere Artikel hierzu finden sich beispielsweise in AESCULAP, Wissenschaftliche Information 22: "Die PLASMAPORE-Beschichtung für die zementlose Verankerung von Gelenkendoprothesen" oder unter "Osteointegration, Oberflächen- und Beschichtungen orthopädischer Implantate für den zementfreien Einsatz" der PI Precision Implants AG. Die vorangegangenen Nachteile eines solchen Flammspritzprozesses konnten jedoch nicht gelöst werden.

Das Problem der durch die Einwirkung von hohen Temperaturen über eine lange Zeit eingeebneten Titanoberfläche kann auch umgangen werden, indem nur lokal und beschränkt Wärme auf die Oberfläche aufgebracht wird. Dies kann beispielsweise durch einzelne Punktschweißungen passieren. Das US-Patent 5,139,528 offenbart hierzu ein Verfahren, bei dem ein mehrlagiges Drahtgeflecht aus Titanfasern auf ein Substrat "gebunden" wird. Nachteilig bei diesem letztgenannten Verfahren mit reduzierter Wärmeeinwirkung ist jedoch, dass die aufgebrachte Schicht keine eigentliche Rauheit im Submikrometerbereich aufweist. Vielmehr besitzt eine solche Beschichtung aufgrund der Regelmäßigkeit des mehrlagigen Drahtgeflechtes aus Titanfasern keine Makrorauhigkeit, d.h. keine Spitzen und Täler, sondern lediglich Poren, die sich von der Oberfläche in die Tiefe erstrecken. Ein wirkungsvolles Anwachsen eines Knochens ist somit auch bei einem solcherart hergestellten Implantat nicht möglich.

Ein weiteres Verfahren, das in der US 5,456,723 offenbart ist, erzielt eine Rauheit im Submikrometerbereich durch ein Ätzen einer Titanoberfläche, die zuvor abrasiv gestrahlt wurde. Eine so hergestellte Oberfläche weist jedoch keine Hohlräume auf, die sich weiter in die Oberfläche hinein erstrecken und in die der Knochen einwachsen könnte.

Gemäß dem vorgenannten lässt sich zusammenfassend festhalten, dass es bisher vielfältige Versuche gab, eine für ein Knocheneinwachsen zufriedenstellend strukturierte Oberfläche auf einem Gelenkersatzimplantat herzustellen. Bislang ist es jedoch lediglich gelungen
- offenporöse Beschichtungen herzustellen, in die der Knochen einwachsen kann, die im Submikrometerbereich jedoch keine topografischen Anreize für die Osteoblastenadhäsion und damit für ein schnelles bzw. gegenüber dem Stand der Technik besseres Knochenanwachsen geben;
- ein Oberflächenverfahren zur Verfügung zu stellen, das eine Rauheit von einigen Mikrometern und eine Submikrometerstruktur für eine bessere Adäsion der Osteoblasten bietet, wobei diese Oberflächen jedoch keine offene Porosität aufweisen, in die der Knochen einwachsen könnte;
- Beschichtungen mit einer großen Rauheit im Bereich von einigen 10 Mikrometern herzustellen, die eine beschränkte Porosität aufweisen, aber wiederum keine eigentliche Submikrometerstruktur besitzen;
- offenporöse Beschichtungen herzustellen, in die der Knochen einwachsen kann und deren Oberfläche eine Submikrometerrauhigkeit aufweist, die jedoch keine ausreichend hohe Makrorauhigkeit aufweisen und sich somit nicht im Knochen "verkrallen" können.

Somit ist es das Ziel der vorliegenden Erfindung, die vorgenannte Lücke zu schließen, wobei es die Aufgabe der Erfindung ist, die Oberfläche eines Gelenkersatzimplantats so zu gestalten, dass die Oberfläche stabile nach außen offene Hohlräume aufweist, die eine Größe haben, die ausreichend ist, dass vaskularisiertes Knochengewebe hineinwachsen kann, wobei die Oberfläche eine sehr gute Biokompatibilität im Sinne einer bioinerten oder leicht bioaktiven Eigenschaft sowie eine gezielt eingestellte Submikrometerrauhigkeit aufweist, die den Osteoblasten als Verankerungspunkte dienen.

Diese Aufgabe wird durch ein Implantat mit einer offenporigen biokompatiblen Oberflächenschicht gemäß Patentanspruch 1, durch ein Verfahren zur Herstellung einer solchen Oberfläche gemäß Patentanspruch 7. sowie durch eine Verwendung gemäß Patentanspruch 25 gelöst.

Insbesondere wird die Aufgabe der Erfindung durch die zur Verfügungstellung eines Implantats, insbesondere Gelenkersatzimplantats, gelöst, das eine erfindungsgemäße offenporige Oberflächenschicht aufweist.

Darüber hinaus wird die Aufgabe durch ein Verfahren zur Herstellung eines offenporigen beschichteten Implantats, insbesondere eines Gelenkersatzimplantats gelöst, das die folgenden Schritte aufweist:
- Aufbringen wenigstens einer Schicht eines biokompatiblen Metalls oder einer Legierung davon auf eine Rohoberfläche des Implantats zur Erzeugung einer Implantatoberfläche,
- Erzeugung einer Oberflächen-Mikrostruktur an der Implantatoberfläche mittels Ätzen der Implantatoberfläche und/oder Aufbringen von feinen biokompatiblen Partikeln an der Implantatoberfläche.

Ein wesentlicher Kerngedanke der Erfindung besteht hierbei darin, dass zunächst als Basis eine Implantatoberfläche geschaffen wird, die in einem zweiten Schritt gezielt mit einer Oberflächenmikrostruktur versehen wird. Dies kann einerseits durch ein in die Tiefe gehen, nämlich ein Ätzen der Implantatoberfläche erfolgen. Die Strukturierung der Oberfläche kann durch das Aufbringen von feinen biokompatiblen Partikeln unterstützt werden. Somit lässt sich auf der biokompatiblen Basisschicht gezielt eine Oberflächenmikrostruktur erstellen, die exakt die geforderten Dimensionen hinsichtlich Mikro- und Makrostruktur erfüllt und dem jeweiligen Knochen- bzw. Gewebetyp angepasst ist bzw. werden kann, so dass ein optimales Einwachsen des Knochens in die Oberflächenschicht gewährleistet ist.

Ein wesentlicher Vorteil hierbei ist, dass eine Relativbewegung zwischen Knochen und Gelenkersatzimplantat minimiert wird, so dass Abriebpartikel im wesentlichen gar nicht erst entstehen. Sollten sich jedoch dennoch Abriebpartikel bilden, was sich bei einer sehr hohen Gelenkbelastung möglicherweise, trotz der optimierten Implantatsverankerungsmöglichkeit gemäß der Erfindung, nicht ausreichend verhindern lässt, so kommt ein zusätzlicher wesentlicher erfindungsgemäßer Vorteil zur Geltung, nämlich der Effekt, dass die im Gelenk entstandenen Abriebpartikel durch ein Labyrinth von Knochen und poröser Oberfläche nur sehr schlecht entlang des Implantats wandern können. Entsprechend langsamer schreitet eine Osteolyse entlang des Implantats fort, was zu einer deutlich verbesserten Lebensdauer und Langzeitverankerung des Gelenkersatzimplantats im Knochen führt.

Gemäß seiner Ausführungsform der Erfindung wird das biokompatible Metall mittels eines Vakuum-Plasma-Spritzverfahrens auf die Rohoberfläche des Implantats aufgebracht. Die Plasmaflamme wird so eingestellt, dass die Titanpartikel zwar leicht angeschmolzen, aber bei einem Aufprall auf das Substrat, nämlich die Implantatrohoberfläche, nur geringfügig kontaktiert werden. Auf diese Weise ist sicher gestellt, dass die Oberfläche nach außen offene Poren behält, eine Glättung der aufgebrachten Oberfläche unterbleibt und eine Makrostruktur erzeugt wird. In diese Makrostruktur, die Poren mit einem durchschnittlichen Durchmesser von 300 Mikrometern aufweist, kann vaskularisiertes Knochengewebe einwachsen. Es sei bereits an dieser Stelle erwähnt, dass der Durchmesser der Poren einstellbar ist. Dies hängt, wie nachfolgend ausgeführt, maßgeblich von der Art des Ausgangsmaterials, respektive der Art des Metalls ab, das auf die Oberfläche des Implantats aufgebracht wird.

Ein alternatives Verfahren zur Aufbringung des biokompatiblen Metalls besteht in einem Pinseln, Streichen, Sprühen oder einer anderen Auftragungstechnik, die geeignet ist, ein fließfähiges Produkt oder eine Paste auf einen Gegenstand aufzubringen.

Eine solche Paste wird hergestellt, indem das biokompatible Metall oder eine Legierung davon zusammen mit einem oder mehreren Bindern und/oder Sinterhilfsmittel(n) gemischt und auf eine fließfähige Konsistenz eingestellt wird. Der Grad der erforderlichen Fließfähigkeit hängt hierbei im wesentlichen von der Oberflächenform des Implantats und von der Wahl der Aufbringungsart der Paste oder Flüssigkeit, die das biokompatible Metall aufweist, ab. Je feiner die Strukturen des Substrats sind, auf das das biokompatible Metall aufgebracht werden soll, desto dünnflüssiger sollte die Paste oder die Flüssigkeit sein, um auch feine Strukturen ausfüllen zu können, ohne jedoch zu Tropfenbildung und Verlaufen zu neigen. Selbiges gilt selbstverständlich, falls das biokompatible Metall mittels Sprühen oder Spritzen aufgetragen wird.

Erfindungsgemäß sind als Binder ein oder mehrere der folgende Substanzen vorgesehen: Karboximethylzellulose, Kollodium, Polyvinylalkohol, Wasser oder ein organisches Lösungsmittel. Ein notwendiges Kriterium bei der Wahl des Binders ist eine im wesentlichen vollständige Entfernungsmöglichkeit aus einer Implantatoberfläche. Die Entfernung kann während der Bearbeitung der Implantatoberfläche erfolgen.

Gemäß einer Ausführungsvariante der Erfindung wird die wenigstens eine auf die Rohoberfläche des Implantats aufgebrachte Schicht gesintert. Vorteilhafterweise wird ein Sintern angewandt, wenn das biokompatible Metall mittels Pinseln, Streichen, Sprühen, Spritzen oder einer anderen derartigen Auftragungstechnik auf die Rohoberfläche des Implantats aufgebracht worden ist. Ein Sintern einer mittels eines Vakuum-Plasma-Spritzverfahrens aufgebrachten biokompatiblen Schicht ist möglich, insbesondere dann, wenn die mit dem Vakuum-Plasma-Spritz-Verfahren aufgetragene Schicht zu große Poren aufweist; d.h. die Porenweite der Implantatoberfläche kann mittels Sintern nachbearbeitet werden.

Das bereits vorerwähnte Sinterhilfsmittel ist erfindungsgemäß ein Sinterhilfsmittel-Metall, das mit dem biokompatiblen Metall oder der Legierung daraus ein tief schmelzendes Eutektikum bildet. Insbesondere wird hierzu Silizium oder Kobalt, vorzugsweise in Elementarform, eingesetzt. Üblicherweise wird das Silizium oder Kobalt als Pulver eingesetzt, das sich mit einem Metallpulver und einem oder mehreren Bindern mischen lässt, so dass eine Paste mit einer homogenen Verteilung der Bestandteile herstellbar und ebenso homogen auf die Rohoberfläche des Implantats aufbringbar ist.

Gemäß einer Ausführungsform der Erfindung wird ein Sintern im Vakuum durchgeführt. Der Vorteil hierbei ist, dass durch das Sintern im Vakuum während der Aufheizphase eine Entbinderung stattfindet, bei der der Binder denaturiert und/oder aus dem System abgezogen wird. Ein weiterer Vorteil besteht darin, dass anstelle eines biokompatiblen Metalls, beispielsweise anstelle von Titanpulver, dessen entsprechende Vorstufe, nämlich das entsprechende Metallhydritpulver, verwendet werden kann. Die Dehydrierung geschieht in diesem Fall ebenfalls in der Aufheizphase des Sinterzyklus.

Eine Sintertemperatur liegt hierbei im Bereich von 800°C bis 1500°C bzw. im Bereich von 950° C bis 1400° C und besonders bevorzugt im Bereich von 1000°C bis 1350°C. Die jeweils geeignete Temperatur hängt maßgeblich von jeweils verwendeten Sinterhilfsmitteln und dessen Verhältnis bezüglich des biokompatiblen Metalls ab und liegt bei Silizium im Bereich von 1295°C bis 1355°C. Bei der Verwendung von Kobalt als Sinterhilfsmittel wird eine bevorzugte Sintertemperatur im Bereich von 1000°C bis 1100°C angewandt.

Erfindungsgemäß wird das biokompatible Metall in Pulverform, insbesondere in Form eines kantigen Pulvers eingesetzt. In vorteilhafter Weise kann so die Bildung einer übermäßig kompakten Implantatoberfläche vermieden werden, da kantige Partikel aufgrund ihrer unregelmäßigen Struktur und Ecken eine dichte (Kugel-)Packung nicht zulassen. Somit ist eine gewisse Basisporosität von vornherein vorgegeben, die hinsichtlich einer Porentiefe der Schichtdicke der Oberflächenschicht auf dem Implantat entspricht.

Die Schichtdicke der offenporigen Oberflächenschicht liegt im Bereich von 0,1 mm bis 2,5 mm, vorzugsweise im Bereich von 0,3 mm bis 1,9 mm und besonders bevorzugt im Bereich von 0,5 mm bis 1,5 mm. Somit ist für ein Verkrallen von Implantat und Knochen eine ausreichende Kontakttiefe vorgesehen.

Vorteilhafterweise weist das biokompatible Metall, das auf die Rohoberfläche des Implantats aufgebracht wird, eine Teilchengröße im Bereich von 50 µm bis 800 µm, vorzugsweise im Bereich von 100 µm bis 650 µm und besonders bevorzugt im Bereich von 200 µm bis 550 µm auf. Somit ist in Abhängigkeit von der Teilchengröße eine Strukturierung der Oberfläche möglich, die in Kombination mit der kantigen Form eines Pulvers, das beispielsweise kantig gemahlen worden ist und/oder in Kombination mit der Schichtdicke der offenporigen Oberflächenschicht die Wahl eines für ein Einwachsen von Knochen optimierten Durchmessers der Poren ermöglicht. Des weiteren sind Hinterschneidungen und Hohlräume innerhalb der Schicht möglich, so dass ein einwachsender Knochen die poröse Oberflächenschicht hintergreifen kann und auf diese Weise eine optimale Verankerung gewährleistet.

Als biokompatibles Metall sind erfindungsgemäß bevorzugt Titan, jedoch auch Zirkon, Niob oder Tantal vorgesehen. Diese Metalle weisen eine ausgezeichnete Bioverträglichkeit auf und gewährleisten die Möglichkeit des Einsatzes unterschiedlicher Metalle, für den Fall, dass ein Metall bei einer Person unvorhersehbare Unverträglichkeiten hervorruft.

Des weiteren kann das biokompatible Metall als Metallhydridpulver eingesetzt werden. Dies ist vorteilhaft, da das Metallhydridpulver eine Vorstufe des eigentlichen Metalls bei dessen Herstellung ist. Eine Dehydrierung geschieht bei einem solchen Pulver beispielsweise in der Aufheizphase des Sinterzyklus, sofern dieser im Vakuum durchgeführt wird.

Gemäß einer Ausführungsform der Erfindung wird das Ätzen der Implantatoberfläche mittels eines Säurebades und/oder mittels Plasmaätzen durchgeführt. Im Falle von Plasmaätzen ist die Verwendung eines Sauerstoffplasmas besonders vorteilhaft. Das jeweils anzuwendende Verfahren hängt von der Art der gewünschten Oberflächenmikrostruktur ab. Während ein Ätzprozeß im Säurebad zur Ausbildung kleiner Ätzgrübchen mit einem Durchmesser von 0,1 µm bis 2,5 µm, vorzugsweise 0,5 µm bis 1,9 µm und besonders bevorzugt in der Größenordnung von 0,8 µm bis 1,5 µm liegt, führt ein Plasmaätzen nicht zu einer Grübchenbildung, sondern bildet eine mikroskopisch feine, eher flächige Aufrauhung der Metalloberfläche aus. Beide Verfahren können nacheinander in Kombination angewandt werden.

Eine weitere Möglichkeit zur Erzeugung einer Oberflächenmikrostruktur an der Implantatoberfläche besteht in dem Aufbringen von feinen biokompatiblen Partikeln an der Implantatoberfläche, wie bereits vorerwähnt. Die feinen biokompatiblen Partikel weisen hierbei eine Korngröße im Bereich von 0,01 µm bis 5 µm, vorzugsweise im Bereich von 0,1 µm bis 3 µm, besonders bevorzugt im Bereich von 0,2 µm bis 1 µm auf. Mit diesem Verfahren findet keine Aufrauung oder Ätzung der Implantatoberfläche statt, sondern sie wird mit den biokompatiblen Partikeln überzogen. Dies geschieht beispielsweise mit Hilfe eines Solgelverfahrens und einem Binder, vorzugsweise auf Silikatbasis. Dieser Partikelbinder verbleibt anders als der vorerwähnte zur Erzeugung einer Paste oder Flüssigkeit an den biokompatiblen Partikeln und ist als solcher ebenfalls biokompatibel. Als Material für die feinen biokompatiblen Partikel eignet sich besonders Titandioxid oder Kalziumphosphat. Es ist jedoch auch möglich, ein anderes geeignetes biokompatibles Material zu verwenden. Die beiden genannten Materialien sind jedoch besonders vorteilhaft, da sie körpereigenen bzw. implantatidentischen oder -ähnlichen Verbindungen entsprechen, die als solche bioverträglich sind.

Des weiteren wird die erfindungsgemäße Aufgabe durch eine offenporige biokompatible Oberflächenschicht gelöst, die auf einer Rohoberfläche des Implantats angeordnet ist, und eine Schichtdicke im Bereich von 0,1 mm bis 2,5 mm, vorzugsweise im Bereich von 0,3 mm bis 1,9 mm, besonders bevorzugt im Bereich von 0,5 mm bis 1,5 mm aufweist und eine Porosität im Bereich von 20 % bis 85 %, vorzugsweise im Bereich von 30 % bis 70 % und besonders bevorzugt im Bereich von 35 % bis 65 % hat. Diese hohe Porosität, die im Einzelfall auch größer als 85 % sein kann, dient einer optimierten Verankerungsmöglichkeit des Knochens in der offenporigen Oberflächenschicht.

Erfindungsgemäß ist als topografischer Anreiz für ein schnelles und optimiertes Knochenanwachsen eine Oberflächenstruktur im Submikrometerbereich bis Mikrometerbereich vorgesehen. Diese besteht einerseits aus Ätzgrübchen mit einem Durchmesser im Bereich von 0,1 µm bis 2,5 µm, vorzugsweise im Bereich von 0,5 µm bis 1,9 µm und besonders bevorzugt im Bereich von 0,8 µm bis 1,5 µm; und andererseits in einer flächigen Aufrauung der offenporigen Oberflächenschicht im Submikrometerbereich sowie im Mikrometerbereich.

Gemäß einer weiteren Ausführungsform der Erfindung weist das Implantat an der Implantatoberfläche biokompatible Partikel, insbesondere aus Titandioxid oder Kalziumphosphat auf. Ein zumindest teilweiser Überzug mit diesen Partikeln ist möglich. Die biokompatiblen Partikel weisen erfindungsgemäß eine Korngröße im Bereich von 0,1 µm bis 5 µm, vorzugsweise im Bereich von 0,1 µm bis 3 µm und besonders bevorzugt im Bereich von 0,2 µm bis 1 µm auf. Sofern dies erwünscht ist, lassen sich verschiedene Größenbereiche miteinander kombinieren. Es ist allerdings hierbei darauf zu achten, dass kein Zusetzen von Poren durch die biokompatiblen Partikel erfolgt, sofern der Durchmesser der nach außen offenen Hohlräume hierbei unter eine Größe sinken würde, der für ein Einwachsen von vaskularisiertem Knochengewebe notwendig ist.

Kombinationen der verschiedenen Oberflächenmikrostrukturen, nämlich Grübchen, flächige Aufrauhung und aufgebrachte biokompatible Partikel sind vorgesehen.

Gemäß einer weiteren Ausführungsform besteht die offenporige Oberflächenschicht des Implantats im wesentlichen aus Titan, Niob, Zirkon,Tantal oder Legierungen davon.

Eine der Aufgabe entsprechende Optimierung der Langzeithaltbarkeit von Knochen-Implantat-Kombinationen wird durch die Verwendung einer erfindungsgemäßen Oberflächenschicht für Hüftschächte, Schalen für Hüftgelenke, Femurkomponenten für einen Kniegelenkersatz, Tibiakomponenten für einen Kniegelenkersatz, Komponenten für einen Schultergelenkersatz, Komponenten für einen Ellbogengelenkersatz, Komponenten für einen Zehengelenkersatz, Komponenten für einen Fingergelenkersatz, für eine Komponente zur Fusion von Wirbelkörpern der Lumbarwirbelsäule, für Komponenten für einen Bandscheibenersatz, für transgingivale Implantatsysteme und für orthodontische, insbesondere kieferorthopädische Implantatsysteme und Zahn(ersatz)implantate erreicht.

Weitere Ausführungsformen der Erfindung ergeben sich aus den Unteransprüchen.

Nachfolgend wird die Erfindung anhand von Ausführungsbeispielen beschrieben.

Ausgehend von einem gemahlenen, kantigen über die Hydridstufe hergestellten Titanpulver wird in einem ersten Schritt eine offenporige Struktur auf die Implantatoberfläche aufgebracht. Das Titanpulver weist dabei eine Korngröße von mindestens 200 µm auf. Die offenporige Schicht selbst hat eine Schichtdicke von 0,5 bis 1,5 mm und eine Porosität von mindestens 40 %. Diese Schicht wird vorzugsweise mittels des Vakuum-Plasma-Spritzverfahrens aufgebracht. Die Plasmaflamme wird hierbei so eingestellt, dass die Titanpartikel zwar leicht angeschmolzen werden, aber bei dem Aufprall auf das Substrat, nämlich die Implantatrohoberfläche, nur geringfügig kompaktiert werden. Die so hergestellte Implantatoberfläche wird anschließend einem Ätzprozeß im Säurebad unterworfen. Durch eine gezielte Prozessführung werden hierbei feine Ätzgrübchen im Durchmesser von ca. 1 µm hergestellt.

Gemäß einem zweiten Ausführungsbeispiel wird die offenporige Schicht über ein Sinterverfahren unter Verwendung eines etwas gröberen, kantigen Titanpulvers mit einer Korngröße von ca. 500 µm hergestellt. Das Titanpulver wird zusammen mit einem Binder, nämlich Wasser und einem Sinterhilfsmittel, nämlich elementarem Siliziumpulver zu einer streichfähigen Paste verrührt und mit einem Pinsel auf die Implantatrohoberfläche aufgebracht. Das elementare Siliziumpulver bildet zusammen mit dem verwendeten Titan ein tiefschmelzendes Eutecticum, das für ein temporäres Flüssigphasensintern ausgenutzt wird. Der Sinterzyklus selbst findet im Vakuum statt und beinhaltet beim Aufheizen eine Entbinderungsphase, bei der das Wasser entfernt wird. Erst dann beginnt der eigentliche Sinterprozeß. Durch die Verwendung von Silizium als Sinterhilfsmittel liegt die Sintertemperatur bei 1330°C. Die so hergestellte, offenporige Schicht wird anschließend einem Ätzprozeß unterworfen, der demjenigen des vorangegangenen Beispiels entspricht.

Gemäß einem dritten Ausführungsbeispiel wird als Sinterhilfsmittel elementares Kobaltpulver anstelle von Siliziumpulver verwendet. Ansonsten gleicht dieses Beispiel dem vorangegangenen zweiten Ausführungsbeispiel. Durch die Verwendung von Kobalt als Sinterhilfsmittel liegt die Sintertemperatur bei ca. 1040°C.

Gemäß einem vierten Ausführungsbeispiel wird der Sinterzyklus im Vakuum ausgeführt. Als biokompatibles Material wird die Vorstufe von Titanpulver, nämlich das entsprechende Titanhydridpulver verwendet. Eine Dehydrierung geschieht bei diesem Pulver in der Aufheizphase des Sinterzyklus. Ansonsten entspricht dieses Ausführungsbeispiel dem Ausführungsbeispiel 2. Auch hier wird Wasser als Binder eingesetzt, das ebenfalls in der Aufheizphase des Sinterzyklus aus dem System entfernt wird.

Statt des Säureätzens kann für die mittels Vakuum-Plasma-Spritz-Verfahren oder mittels Sintern hergestellten Schichten ein Plasmaätzen treten. Die Ätzung wird vorzugsweise im Sauerstoffplasma durchgeführt. Dabei tritt keine Grübchenbildung auf, sondern es wird eine mikroskopisch feine, eher flächige Aufrauung der Titanoberfläche bewirkt.

An dieser Stelle sei betont, dass selbstverständlich die Implantatrohoberfläche vor der Aufbringung von zumindest einer Schicht des biokompatiblen Metalls ebenfalls geätzt werden kann. Ebenso ist die Aufbringung mehrerer Schichten des biokompatiblen Metalls möglich. Dies richtet sich in erster Linie nach der gewünschten Schichtdicke und der gewünschten Strukturierung der porösen Oberflächenschicht sowie der Form der Poren. So ist es möglich, beispielsweise hinsichtlich des Porendurchmessers einen Gradienten zu erzeugen, so dass sich die Poren von der äußeren Oberfläche hin zur Implantatrohoberfläche verjüngen; ebenso ist eine Erweiterung des Porendurchmessers hin zur Implantatrohoberfläche möglich. Auf diese Weise ist es vorteilhafterweise möglich, dass eine ankerartige Osteoblastenansammlung mit einer Verbindung zum Knochen innerhalb der porösen Oberflächenschicht ausgebildet wird. Mithin wäre die poröse Oberflächenschicht in Richtung der Implantatrohoberfläche hinterschnitten, so dass eine Ankerwirkung des Knochens an der porösen Oberflächenschicht optimiert ist.

Gemäß eines fünften Ausführungsbeispiels wird die mittels Vakuum-Plasma-Spritz-verfahren oder mittels Sintern hergestellte Schicht nicht geätzt, sondern mit feinen biokompatiblen Partikeln überzogen. Als solche feinen Partikel werden in zwei Varianten entweder Titandioxidpulver oder Kalziumphoshatpulver verwendet. Die Korngröße beträgt in beiden Fällen 1 µm. Diese Pulver werden im Sol-Gel-Verfahren mit einem Binder auf Silikatbasis aufgebracht.

An dieser Stelle sei darauf hingewiesen, dass alle oben beschriebenen Teile für sich alleine gesehen und in jeder Kombination als erfindungswesentlich beansprucht werden. Abänderungen hiervon sind dem Fachmann geläufig.

## Patentansprüche

1. Implantat, insbesondere Gelenkersatzimplantat, mit einer offenporigen biokompatiblen Oberflächenschicht, die auf einer Rohoberfläche des Implantats angeordnet ist,
**dadurch gekennzeichnet, dass**
- die Schichtdicke der offenporigen Oberflächenschicht im Bereich von 0,1 mm bis 2,5 mm, vorzugsweise im Bereich von 0,3 mm bis 1,9 mm und besonders bevorzugt im Bereich von 0,5 mm bis 1,5 mm liegt,
- eine Porosität der offenporigen Oberflächenschicht im Bereich von 20 % bis 85 %, vorzugsweise im Bereich von 30 % bis 70 % und besonders bevorzugt im Bereich von 35 % bis 65 % liegt, und
- die offenporige Oberflächenschicht eine flächige Aufrauung im Submikrometerbereich aufweist.

2. Implantat nach Anspruch 1,
**dadurch gekennzeichnet, dass**
die offenporige Oberflächenschicht Grübchen, insbesondere Ätzgrübchen, mit einem Durchmesser im Bereich von 0,1 µm bis 2,5 µm, vorzugsweise im Bereich von 0,5 µm bis 1,9 µm und besonders bevorzugt im Bereich von 0,8 µm bis 1,5 µm aufweist.

3. Implantat nach einem der vorhergehenden Ansprüche,
**dadurch gekennzeichnet, dass**
auf der Implantatoberfläche biokompatible Partikel, insbesondere aus Titandioxid oder Calciumphosphat, angeordnet sind.

4. Implantat nach Anspruch 3,
**dadurch gekennzeichnet, dass**
die biokompatiblen Partikel eine Korngröße im Bereich von 0,01 µm bis 5 µm, vorzugsweise im Bereich von 0,1 µm bis 3 µm und besonders bevorzugt im Bereich von 0,2 µm bis 1 µm aufweisen.

5. Implantat nach einem der Ansprüche 1 oder 2,
**dadurch gekennzeichnet, dass**
die offenporige Oberflächenschicht im wesentlichen aus Titan, Zirkon, Niob oder Tantal besteht.

6. Implantat nach einem der Ansprüche 1 bis 3,
**dadurch gekennzeichnet, dass**
die offenporige Oberflächenschicht gesintert ist.

7. Verfahren zur Herstellung eines offenporigen beschichteten Implantats gemäß Anspruch 1, insbesondere Gelenkersatzimplantats,
**gekennzeichnet durch**
die folgenden Schritte:
- Aufbringen wenigstens einer Schicht eines biokompatiblen Metalls oder einer Legierung davon auf eine Rohoberfläche des Implantats zur Erzeugung einer Implantatoberfläche,
- Erzeugung einer Oberflächen-Mikrostruktur an der Implantatoberfläche mittels Ätzen der Implantatoberfläche und/oder Aufbringen von feinen biokompatiblen Partikeln an der Implantatoberfläche.

8. Verfahren nach Anspruch 7,
**dadurch gekennzeichnet, dass**
das biokompatible Metall mittels eines Vakuum-Plasma-Spritzverfahrens aufgebracht wird.

9. Verfahren nach Anspruch 7,
**dadurch gekennzeichnet, dass**
das biokompatible Metall mittel Pinseln, Streichen, Sprühen oder dergleichen Auftragungstechniken aufgebracht wird.

10. Verfahren nach einem der Ansprüche 7 bis 9, insbesondere nach Anspruch 9,
**dadurch gekennzeichnet, dass**
die wenigstens eine auf die Rohoberfläche des Implantats aufgebrachte Schicht gesintert wird.

11. Verfahren nach Anspruch 10,
**dadurch gekennzeichnet, dass**
Binder und/oder Sinterhilfsmittel verwendet werden.

12. Verfahren nach Anspruch 11,
**dadurch gekennzeichnet, dass**
als Sinterhilfsmittel ein Sinterhilfsmittel-Metall eingesetzt wird, das mit dem biokompatiblen Metall oder der Legierung daraus ein tiefschmelzendes Eutektikum bildet, insbesondere Silizium oder Kobalt, vorzugsweise in elementarer Pulverform.

13. Verfahren nach einem der Ansprüche 10 bis 12,
**dadurch gekennzeichnet, dass**
ein Sintern im Vakuum durchgeführt wird.

14. Verfahren nach einem der Ansprüche 10 bis 13,
**dadurch gekennzeichnet, dass**
das Sintern eine Entbinderungs- und/oder Dehydrierungsphase umfasst.

15. Verfahren nach einem der Ansprüchen 10 bis 14,
**dadurch gekennzeichnet, dass**
eine Sintertemperatur im Bereich von 800°C bis 1500°C, vorzugsweise im Bereich von 950°C bis 1400°C und besonders bevorzugt im Bereich von 1000°C bis 1350°C angewandt wird.

16. Verfahren nach einem der Ansprüche 7 bis 15,
**dadurch gekennzeichnet, dass**
das biokompatible Metall in Pulverform, insbesondere Form eines kantigen Pulvers eingesetzt wird.

17. Verfahren nach einem der Ansprüche 7 bis 16,
**dadurch gekennzeichnet, dass**
eine Schichtdicke der offenporigen Oberflächenschicht im Bereich von 0,1 mm bis 2,5 mm, vorzugsweise im Bereich von 0,3 mm bis 1,9 mm und besonders bevorzugt im Bereich von 0,5 mm bis 1,5 mm erzeugt wird.

18. Verfahren nach einem der Ansprüche 7 bis 17,
**dadurch gekennzeichnet, dass**
das biokompatible Metall, das auf die Rohoberfläche des Implantats aufgebracht wird, eine Teilchengröße im Bereich von 50 µm bis 800 µm, vorzugsweise im Bereich von 100 µm bis 650 µm und besonders bevorzugt im Bereich von 200 µm bis 550 µm aufweist.

19. Verfahren nach einem der Ansprüche 7 bis 18,
**dadurch gekennzeichnet, dass**
das biokompatible Metall Titan, Zirkon, Niob oder Tantal ist.

20. Verfahren nach einem der Ansprüche 9 bis 19,
**dadurch gekennzeichnet, dass**
das biokompatible Metall als Metallhydridpulver eingesetzt wird.

21. Verfahren nach einem der Ansprüche 7 bis 20,
**dadurch gekennzeichnet, dass**
das Ätzen der Implantatoberfläche mittels Säure(bad)ätzen und/oder mittels Plasmaätzen, insbesondere Sauerstoffplasma durchgeführt wird.

22. Verfahren nach einem der Ansprüche 7 bis 21,
**dadurch gekennzeichnet, dass**
die feinen biokompatiblen Partikel eine Korngröße im Bereich von 0,01 µm bis 5 µm, vorzugsweise im Bereich von 0,1 µm bis 3 µm und besonders bevorzugt im Bereich von 0,2 µm bis 1 µm aufweisen.

23. Verfahren nach einem der Ansprüche 7 bis 22,
**dadurch gekennzeichnet, dass**
die feinen biokompatiblen Partikel im Solgelverfahren mit einem Binder, vorzugsweise auf Silikatbasis, aufgebracht werden.

24. Verfahren nach einem der Ansprüche 7 bis 23,
**dadurch gekennzeichnet, dass**
als Material für die feinen biokompatiblen Partikel Titandioxid, Calciumphosphat oder ein anderes biokompatibles Material verwendet wird.

25. Verwendung einer auf einem Implantat befindlichen Oberflächenschicht gemäß einem der Ansprüche 1 bis 6,
für Hüftschäfte, Schalen für Hüftgelenke, Femurkomponenten für einen Kniegelenkersatz, Tibiakomponenten für einen Kniegelenkersatz, Komponenten für einen Schultergelenkersatz, Komponenten für einen Ellbogengelenkersatz, Komponenten für einen Zehengelenkersatz, Komponenten für einen Fingergelenkersatz, für eine Komponente zur Fusion von Wirbelkörpern der Lumbarwirbelsäule, für Komponenten für einen Bandscheibenersatz, für transgingivale Implantatsysteme, für orthodontische Implantatsysteme und Zahn(ersatz)implantate.

## Claims

1. Implant, in particular, a joint-replacement implant, with an open-pore, bio-compatible surface layer, which is disposed on an untreated surface of the implant,
**characterised in that**
- the layer thickness of the open-pore surface layer is disposed within the range from 0.1 mm to 2.5 mm, preferably within the range from 0.3 mm to 1.9 mm and by particular preference within the range from 0.5 mm to 1.5 mm,
- a porosity of the open-pore surface layer is disposed within the range from 20% to 85%, preferably within the range from 30% to 70% and by particular preference within the range from 35% to 65%, and
- open-pore surface layer provides a surface roughening in the sub-micrometre range.

2. Implant according to claim 1,
**characterised in that**
the open-pore surface layer provides pits, in particular etching pits, with a diameter within the range from 0.1 µm to 2.5 µm, preferably within the range from 0.5 µm to 1.9 µm and by particular preference within the range from 0.8 µm to 1.5 µm.

3. Implant according to any one of the preceding claims,
**characterised in that**
bio-compatible particles, in particular, made of titanium dioxide or calcium phosphate, are disposed on the implant surface.

4. Implant according to claim 3,
**characterised in that**
the bio-compatible particles provide a grain size within the range from 0.01 µm to 5 µm, preferably within the range from 0.1 µm to 3 µm, and by particular preference within the range from 0.2 µm to 1 µm.

5. Implant according to anyone of claims 1 or 2,
**characterised in that**
the open-pore surface layer consists substantially of titanium, zirconium, niobium or tantalum.

6. Implant according to any one of claims 1 to 3,
**characterised in that**
the open-pore surface layer is sintered.

7. Method for the manufacture of an open-pore, coated implant according to claim 1, in particular, a joint-replacement implant,
**characterised by**
the following steps:
- application of at least one layer of bio-compatible metal or an alloy thereof to an untreated surface of the implant in order to form an implant surface,
- formation of a surface micro-structure on the implant surface by means of etching the implant surface and/or applying fine, bio-compatible particles to the implant surface.

8. Method according to claim 7,
**characterised in that**
the bio-compatible metal is applied by means of a vacuum-plasma spraying process.

9. Method according to claim 7,
**characterised in that**
the bio-compatible metal is applied by means of brushstrokes, painting, spraying or similar application techniques.

10. Method according to any one of the preceding claims 7 to 9, in particular, according to claim 9,
**characterised in that**
the at least one layer applied to the untreated surface of the implant is sintered.

11. Method according to claim 10,
**characterised in that**
auxiliary binding and/or sintering agents are used.

12. Method according to claim 11,
**characterised in that**
a metallic auxiliary-sintering agent, which forms a low-melting-point eutectic composition, with the biocompatible metal or the alloy thereof, in particular, silicon or cobalt, preferably in elemental, powdered form, is used as an auxiliary-sintering agent.

13. Method according to anyone of claims 10 to 12,
**characterised in that**
a sintering is implemented under vacuum.

14. Method according to any one of claims 10 to 13,
**characterised in that**
the sintering comprises a debinding and/or dehydration phase.

15. Method according to anyone of claims 10 to 14,
**characterised in that**
a sintering temperature within the range from 800°C to 1500°C, preferably within the range from 950°C to 1400°C and by particular preference within the range from 1000°C to 1350°C is used.

16. Method according to anyone of claims 7 to 15,
**characterised in that**
the bio-compatible metal is used in powdered form, in particular, in the form of an angular powder.

17. Method according to anyone of claims 7 to 16,
**characterised in that**
a layer thickness of the open-pore surface layer is formed within the range from 0.1 mm to 2.5 mm, preferably within the range from 0.3 mm to 1.9 mm and by particular preference within the range from 0.5 mm to 1.5 mm.

18. Method according to anyone of claims 7 to 17,
**characterised in that**
the bio-compatible metal, which is applied to the untreated surface of the implant, provides a particle size within the range from 50 µm to 800 µm, preferably within the range from 100 µm to 650 µm and by particular preference within the range from 200 µm to 550 µm.

19. Method according to any one of claims 7 to 18,
**characterised in that**
the bio-compatible metal is titanium, zirconium, niobium or tantalum.

20. Method according to anyone of claims 9 to 19,
**characterised in that**
the bio-compatible metal is used as a metal-hydride powder.

21. Method according to anyone of claims 7 to 20,
**characterised in that**
the etching of the implant surface is implemented by means of acid-(bath) etching and/or by means of plasma etching, in particular, oxygen plasma.

22. Method according to anyone of claims 7 to 21,
**characterised in that**
the fine bio-compatible particles provide a grain size within the range from 0.01 µm to 5 µm, preferably within the range from 0.1 µm to 3 µm and by particular preference within the range from 0.2 µm to 1 m.

23. Method according to anyone of claims 7 to 22,
**characterised in that**
the fine, bio-compatible particles are applied in the sol-gel process with a binder, preferably based on silicate.

24. Method according to anyone of claims 7 to 23,
**characterised in that**
titanium dioxide, calcium phosphate or another biocompatible material is used as the material for the fine, bio-compatible particles.

25. Use of a surface layer disposed on an implant according to any one of claims 1 to 6,
for hip shafts, cups for hip joints, femur components for a knee-joint replacement, tibia components for a knee-joint replacement, components for a shoulder-joint replacement, components for an elbow-joint replacement, components for a toe-joint replacement, components for a finger-joint replacement, for a component for fusing vertebral bodies of the lumbar spine, for components for an intervertebral disk replacement, for transgingival implant systems, for orthodontic implant systems and tooth (replacement) implants.

## Revendications

1. Implant, notamment implant d'articulation artificielle, avec un revêtement biocompatible à pores ouverts, appliqué sur une surface brute de l'implant,
**caractérisé en ce que**
- l'épaisseur du revêtement à pores ouverts est comprise entre 0,1 mm et 2,5 mm, de préférence entre 0,3 mm et 1,9 mm et de manière particulièrement préférentielle entre 0,5 mm et 1,5 mm,
- la porosité du revêtement à pores ouverts est comprise entre 20% et 85%, de préférence entre 30% et 70% et de manière particulièrement préférentielle entre 35 et 65%, et
- le revêtement à pores ouverts présente une rugosité mince d'ordre submicroscopique.

2. Implant selon la revendication 1,
**caractérisé en ce que**
le revêtement à pores ouverts présente des piqûres, notamment des piqûres de corrosion, d'un diamètre compris entre 0,1 µm et 2,5 µm, de préférence entre 0,5 µm et 1,9 µm et de manière particulièrement préférentielle entre 0,8 µm et 1,5 µm.

3. Implant selon l'une quelconque des revendications précédentes,
**caractérisé en ce que**
des particules biocompatibles, notamment de dioxyde de titane ou de phosphate de calcium, sont appliquées à la surface de l'implant.

4. Implant selon la revendication 3,
**caractérisé en ce que**
les particules biocompatibles présentent une granulométrie de l'ordre de 0,01 µm à 5 µm, de préférence de l'ordre de 0,1 µm à 3 µm et de manière particulièrement préférentielle de l'ordre de 0,2 µm à 1 µm.

5. Implant selon la revendication 1 ou la revendication 2,
**caractérisé en ce que**
le revêtement à pores ouverts se compose essentiellement de titane, de zirconium, de niobium ou de tantale.

6. Implant selon l'une quelconque des revendications 1 à 3,
**caractérisé en ce que**
le revêtement à pores ouverts est aggloméré par frittage.

7. Procédé de fabrication d'un implant à revêtement à pores ouverts selon la revendication 1, notamment un implant d'articulation artificielle
**caractérisé par**
les étapes suivantes :
- application d'au moins une couche d'un métal biocompatible ou d'un alliage de celui-ci sur une surface brute de l'implant en vue d'obtenir une surface d'implant,
- production d'une microstructure superficielle à la surface de l'implant par attaque de ladite surface de l'implant et/ou application de fines particules biocompatibles à la surface de l'implant.

8. Procédé selon la revendication 7,
**caractérisé en ce que**
le métal biocompatible est appliqué selon un procédé de projection au plasma sous vide.

9. Procédé selon la revendication 7,
**caractérisé en ce que**
le métal biocompatible est appliqué par badigeonnage, enduction, pulvérisation ou autres techniques d'application similaires.

10. Procédé selon l'une quelconque des revendications 7 à 9, notamment selon la revendication 9,
**caractérisé en ce que**
la ou les couches appliquées sur la surface brute de l'implant sont agglomérées par frittage.

11. Procédé selon la revendication 10,
**caractérisé en ce que**
il est employé des agents liants et/ou des agents de frittage.

12. Procédé selon la revendication 11,
**caractérisé en ce que**
l'agent de frittage utilisé est un métal de frittage qui forme avec le métal biocompatible ou l'alliage de ce métal un eutectique à bas point de fusion, notamment du silicium ou du cobalt, de préférence sous une forme élémentaire pulvérulente.

13. Procédé selon l'une quelconque des revendications 10 à 12,
**caractérisé en ce que**
il est effectué un frittage sous vide.

14. Procédé selon l'une quelconque des revendications 10 à 13,
**caractérisé en ce que**
l'opération de frittage comprend une phase de déliantage et/ou de déshydrogénation.

15. Procédé selon l'une quelconque des revendications 10 à 14,
**caractérisé en ce que**
il est appliqué une température de frittage comprise entre 800 °C et 1500°C, de préférence entre 950 °C et 1400 °C et de manière particulièrement préférentielle entre 1000 °C et 1350 °C.

16. Procédé selon l'une quelconque des revendications 7 à 15,
**caractérisé en ce que**
le métal biocompatible est mis en oeuvre sous forme pulvérulente, notamment sous la forme d'une poudre irrégulière.

17. Procédé selon l'une quelconque des revendications 7 à 16,
**caractérisé en ce que**
il est obtenu une épaisseur de revêtement à pores ouverts comprise entre 0,1 mm et 2,5 mm, de préférence entre 0,3 mm et 1,9 mm et de manière particulièrement préférentielle entre 0,5 mm et 1,5 mm.

18. Procédé selon l'une quelconque des revendications 7 à 17,
**caractérisé en ce que**
le métal biocompatible appliqué sur la surface brute de l'implant présente une granulométrie comprise entre 50 µm et 800 µm, de préférence entre 100 µm et 650 µm et de manière particulièrement préférentielle entre 200 µm et 550 µm.

19. Procédé selon l'une quelconque des revendications 7 à 18,
**caractérisé en ce que**
le métal biocompatible est le titane, le zirconium, le niobium ou le tantale.

20. Procédé selon l'une quelconque des revendications 9 à 19,
**caractérisé en ce que**
le métal biocompatible est mis en oeuvre sous la forme d'une poudre d'hydrure métallique.

21. Procédé selon l'une quelconque des revendications 7 à 20,
**caractérisé en ce que**
l'attaque de la surface de l'implant est effectuée par attaque (en bain) acide et/ou par gravure au plasma, notamment au plasma d'oxygène.

22. Procédé selon l'une quelconque des revendications 7 à 21, **caractérisé en ce que**
les fines particules biocompatibles ont une granulométrie comprise entre 0,01 µm et 5 µm, de préférence entre 0,1 µm et 3 µm et de manière particulièrement préférentielle entre 0,2 µm et 1 µm.

23. Procédé selon l'une quelconque des revendications 7 à 22,
**caractérisé en ce que**
les fines particules biocompatibles sont appliquées selon le procédé sol-gel avec un liant, de préférence à base de silicate.

24. Procédé selon l'une quelconque des revendications 7 à 23,
**caractérisé en ce que**
le matériau employé pour les fines particules biocompatibles est le dioxyde de titane, le phosphate de calcium ou un autre matériau biocompatible.

25. Utilisation d'un revêtement situé sur un implant selon l'une quelconque des revendications 1 à 6,
pour des tiges fémorales, des cupules pour articulation de la hanche, des composantes fémorales pour prothèse du genou, des composantes tibiales pour prothèse du genou, des composantes pour prothèse de l'épaule, des composantes pour prothèse du coude, des composantes pour prothèse d'articulation du doigt de pied, des composantes pour prothèse d'articulation du doigt, une composante pour la fusion de corps vertébraux du rachis lombaire, des composantes pour prothèse de disque intervertébral, des systèmes d'implant transgingival, des systèmes d'implant orthodontique et des implants (de prothèses) dentaires.
